(19) **Europäisches Patentamt / European Patent Office / Office européen des brevets**

(11) **EP 4 760 728 A1**

(12) **EUROPEAN PATENT APPLICATION**
published in accordance with Art. 153(4) EPC

(43) Date of publication:
17.06.2026 Bulletin 2026/25

(21) Application number: 25777783.9

(22) Date of filing: 27.03.2025

(51) International Patent Classification (IPC):
$G16C\ 10/00^{(2019.01)}$     $G16C\ 20/70^{(2019.01)}$
$G16C\ 60/00^{(2019.01)}$     $G06N\ 3/08^{(2023.01)}$

(52) Cooperative Patent Classification (CPC):
G06F 30/27; G06N 3/02; G06N 3/08; G06N 20/00;
G16C 10/00; G16C 20/70; G16C 60/00

(86) International application number:
PCT/KR2025/003954

(87) International publication number:
WO 2025/206772 (02.10.2025 Gazette 2025/40)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 29.03.2024 JP 2024056799

(71) Applicant: LG Energy Solution, Ltd.
Seoul 07335 (KR)

(72) Inventors:
• KIM, Min-Jung
Daejeon 34122 (KR)
• UMEZAWA, Naoto
Yokohama-shi, Kanagawa 220--0011 (JP)
• NARIMATSU, Eiichiro
Yokohama-shi, Kanagawa 220--0011 (JP)
• MATSUBARA, Keiko
Yokohama-shi, Kanagawa 220--0011 (JP)

• OKUSHIMA, Shun
Yokohama-shi, Kanagawa 220--0011 (JP)
• KANG, Beom-Chang
Daejeon 34122 (KR)
• CHOI, Young-Cheol
Daejeon 34122 (KR)
• HAH, Hoe-Jin
Daejeon 34122 (KR)
• KIM, Young-Bok
Daejeon 34122 (KR)
• KIM, Su-Hwan
Daejeon 34122 (KR)
• SHIM, Sung-Geun
Daejeon 34122 (KR)
• HWANG, Jee-Min
Daejeon 34122 (KR)

(74) Representative: Goddar, Heinz J.
Boehmert & Boehmert
Anwaltspartnerschaft mbB
Pettenkoferstrasse 22
80336 München (DE)

(54) **TRAINING APPARATUS, ESTIMATION APPARATUS, TRAINING METHOD, ESTIMATION METHOD, AND PROGRAM**

(57) The present disclosure is designed to improve the accuracy of total energy estimation by neural network potential (NNP). An aspect of the present disclosure provides a learning apparatus including a control unit to carry out learning of an estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential, and a 3D wave function of a u-th atomic orbital of each of other atoms in the system within a predetermined range of distances from the evaluation target in descending order of occupied energy potential, wherein in the learning, the estimation model is updated to reduce a difference between the sum of results of executing the estimation model for each atom in the system and the energy of the system estimated by density functional theory.

(Cont. next page)

FIG. 1

**Description**

This application is based on and claims priority from Japan Patent Application No. 2024-056799 filed on March 29, 2024 with the Japan Intellectual Property Office, the disclosure of which is incorporated herein in its entirety by reference.

TECHNICAL FIELD

The present disclosure relates to a learning apparatus, an estimation apparatus, a learning method, an estimation method and a program.

BACKGROUND

Density functional theory is used to investigate the properties of materials in order to develop new drugs or improve the performance of semiconductor devices. However, density functional theory calculations have high computational costs and limited cell size (the repeating unit lattice size of a crystal) of about 200 atoms at which the composition can be investigated, thereby failing to study the properties of materials of larger systems. For example, to reproduce the composition of solid electrolytes added with a trace amount of dopants, the required cell size is 500 atoms or more, making it harder to sufficiently understand the materials.

Accordingly, many studies are being conducted to reduce the computational costs by building neural network-based interatomic potential models (Patent Literature 1 and Non-Patent Literatures 1 to 3). Here, training data is generated from density functional theory calculations of relatively small systems, and a generalized mathematical model known as neural network potential (NNP) is created to accurately reproduce the results. The use of NNP significantly reduces the computational costs of interatomic forces and total energy, compared to density functional theory calculations. By this reason, it is applied to molecular dynamics calculations of large systems.

<Related Art Literature>

[Patent Literature]

Patent Literature 1: US Patent Publication No. 2022/0207393

[Non-Patent Literature]

Non-Patent Literature 1: J. Behler and M. Parrinello, "Generalized Neural-Network Representation of High-Dimensional Potential-Energy Surfaces", Phys. Rev. Lett. Vol. 98, pp. 146401(2007)
Non-Patent Literature 2: AP Bartok et al. "Gaussian Approximation Potentials: The Accuracy of Quantum Mechanics, without the Electrons", Phys. Rev. Lett. Vol. 104, pp. 136403(2010)
Non-Patent Literature 3: AP Thompson, et al. "Spectral neighbor analysis method for automated generation of quantum-accurate interatomic potentials." Journal of Computational Physics 285(2015): 316-330.

DISCLOSURE

Technical Problem

The neural network potentials (NNPS) proposed so far have low accuracy of total energy prediction, making it difficult to analyze the properties of materials involving calculating total energy such as formation energy or elastic properties of the materials.

In view of the above, the present invention is intended to provide a technique for improving the estimation accuracy of total energy by neural network potential (NNP).

Technical Solution

An aspect of the present disclosure provides a learning apparatus including a control unit to carry out learning of an estimation model which is a mathematical model for energy estimation of an evaluation target atom based on an evaluation target feature quantity indicating a first sum to a u-th sum, wherein the u-th sum is a sum of a three-dimensional (3D) wave function representing a u-th atomic orbital in descending order of occupied energy potential among atomic orbitals of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential, wherein in the learning, the estimation model is updated to reduce a difference between the sum of results of executing the estimation model for each atom in the system and the energy of the system estimated by density functional theory for the system.

An aspect of the present disclosure provides an estimation apparatus including an estimation unit to carry out estimation by a trained estimation model acquired by a learning apparatus, the learning apparatus including a control unit to carry out learning of the estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential, wherein in the learning, the estimation model is updated to reduce a difference between the sum of results of executing the estimation model for each atom in the system and the energy of the system estimated by density functional theory for the system.

An aspect of the present disclosure provides a learning method performed by a learning apparatus including a control unit to carry out learning of an estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential,

wherein in the learning, the estimation model is updated to reduce a difference between the sum of results of executing the estimation model for each atom in the system and the energy of the system estimated by density functional theory for the system, and
wherein the learning method includes a control step of, by the control unit, carrying out the learning of the estimation model.

An aspect of the present disclosure provides an estimation method performed by an estimation apparatus including an estimation unit to carry out estimation by a trained estimation model acquired by a learning apparatus, the learning apparatus including a control unit to carry out learning of the estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential,

wherein in the learning, the estimation model is updated to reduce a difference between the sum of results of executing the estimation model for each atom in the system and the energy of the system estimated by density functional theory for the system, and
wherein the estimation method includes an estimation step of, by the estimation unit, carrying out the estimation by the trained estimation model.

An aspect of the present disclosure provides a program for causing a computer to act as the learning apparatus.
An aspect of the present disclosure provides a program for causing a computer to act as the estimation apparatus.

Advantageous Effects

According to the present disclosure, it may be possible to improve the accuracy of total energy estimation by neural network potential (NNP).

BRIEF DESCRIPTION OF THE DRAWINGS

FIG. 1 is a diagram illustrating an estimation system according to an embodiment.
FIG. 2 is a diagram showing an example of hardware configuration of a learning apparatus according to an embodiment.
FIG. 3 is a flowchart showing an example of a flow of processing executed by a learning apparatus according to an embodiment.
FIG. 4 is a diagram showing an example of hardware configuration of an estimation apparatus according to an embodiment.
FIG. 5 is a flowchart showing an example of a flow of processing executed by an estimation apparatus according to an embodiment.
FIG. 6 is a first diagram showing an example of experimental results according to an embodiment.
FIG. 7 is a second diagram showing an example of experimental results according to an embodiment.

BEST MODE

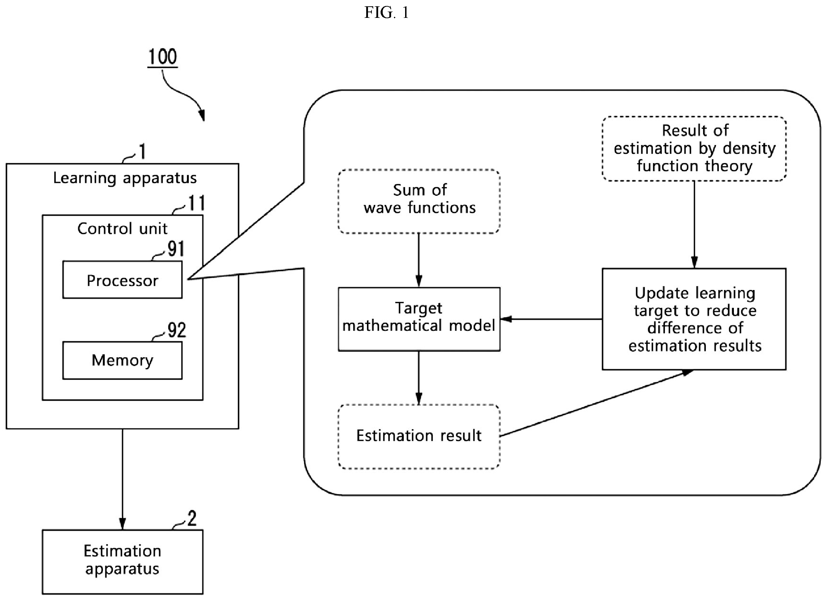

FIG. 1 is a diagram illustrating an estimation system 100 according to an embodiment. The estimation system 100 includes a learning apparatus 1 and an estimation apparatus 2. The estimation apparatus 2 carries out estimation using a trained mathematical model acquired by the learning apparatus 1. Specifically, the trained mathematical model acquired by the learning apparatus 1 is referred to as a trained estimation model as described below. The trained estimation model is a type of neural network potential (NNP).

The learning apparatus 1 includes a control unit 11 having a processor 91, for example, a Central Processing Unit (CPU), a Graphics Processing Unit (GPU) or a Neural Network Processing Unit (NPU) and a memory 92 connected via a bus. For example, the control unit 11 carries out learning processing. The learning processing is processing for acquiring the trained estimation model by training the estimation model which is a target mathematical model, until a predetermined condition for the end of learning (hereinafter referred to as "learning end condition") is met.

The estimation model is a mathematical model that estimates the energy of an evaluation target atom based on an evaluation target feature quantity. The evaluation target feature quantity is the quantity indicating a first sum to a u-th sum (u is an integer that is equal to or larger than 1 and is equal to or smaller than U, and U is an integer of 1 or greater). The u-th sum is the sum of a three-dimensional (3D) wave function representing a u-th central atomic orbital and a 3D wave function representing a u-th peripheral atomic orbital.

The u-th central atomic orbital is the atomic orbital of the evaluation target atom located within a system to be analyzed (hereinafter referred to as "analysis target system") including one or more atoms, and is the u-th atomic orbital in descending order of occupied potential energy.

The u-th peripheral atomic orbital is the atomic orbital of each of surrounding atoms within a predetermined range of distances from the evaluation target atom, and is the u-th atomic orbital in descending order of occupied potential energy. The surrounding atom is an atom other than the evaluation target among the atoms in the analysis target system.

For example, when the analysis target system is $Li_6PS_5Cl$ and the evaluation target atom is S, the surrounding atom is, for example, Li, P and Cl. In this case, when u = 1, the u-th central atomic orbital is a 3p orbital, the u-th central atomic orbital of one of the surrounding atoms, Li, is a 2s orbital, and the u-th central atomic orbital of the other surrounding atoms, P and Cl, is a 3p orbital.

<Example of evaluation target feature quantity>

The evaluation target feature quantity is, for example, a vector represented by Equation (1) below. Meanwhile, in Equation (1) where u=2, the number of elements in the vector is 2, but there is no need to rely on the value of u, and the number of elements is not limited to 2. The dimension of the vector indicating the evaluation target feature quantity is u. Where u = 1, the evaluation target feature quantity is scalar.

【Equation 1】

$$G_i(\mathbf{R}) = \left[ G_i^{(n,l)_i}(\mathbf{R}), G_i^{(n',l')_i}(\mathbf{R}) \right]$$

【Equation 2】

$$G_i^{(n,l)_i}(\mathbf{R}) = \rho_i^{(n,l)_i}(R) + \sum_{j=1 \neq i}^{N} \rho_j^{(n,l)_j}\left(|r_i + \mathbf{R} - r_j|\right)$$

Here, $(n,l)_i$ indicates the principal quantum number n and the azimuthal quantum number l of an i-th atom. N denotes the number of atoms located within a sphere of a predetermined radius from the i-th atom at the center. The predetermined radius is, for example, 7Å. $\rho_i$ denotes the wave function of the i-th atom. $\rho_j$ denotes the wave function of a j-th atom. The j-th atom is a different atom from the i-th atom. R denotes the position in 3D space. $r_i$ denotes the position of the i-th atom in 3D space.

For example, the evaluation target feature quantity of a sulfur atom where u=2 is represented by Equation (3) below.

【Equation 3】

$$G_i(\mathbf{R}) = \left[ G_i^{3s}(\mathbf{R}), G_i^{3p}(\mathbf{R}) \right]$$

In the learning, for each atom in the analysis target system, the energy of the evaluation target atom is estimated based on the evaluation target feature quantity of each atom to be evaluated. The energy estimation processing of the evaluation target atom based on the evaluation target feature quantity of the evaluation target atom is executed by the estimation model.

Additionally, in the learning, the estimation model is updated to reduce a difference between the sum (i.e., total energy) of the estimated energies (i.e., the results of executing the estimation model) and the energy of the analysis target system estimated by the density functional theory.

Accordingly, in the training of the estimation model, a pair of information indicating the evaluation target feature quantity for each atom in the analysis target system (hereinafter referred to as 'training input data') and information indicating the estimated energy of the analysis target system by the density functional theory (hereinafter referred to as 'training label') is used in the training. The training label is a label indicating a correct answer. Accordingly, the pair is so-called labelled training data.

The density functional theory for the analysis target system may be implemented by the control unit 11 during learning processing or may be completed by a device other than the learning apparatus 1 before learning processing, and may be implemented by a device other than the learning apparatus 1 in synchronization with the learning processing by the control unit 11.

Meanwhile, the 3D wave function may be, for example, a 3D radial wave function.

Meanwhile, the analysis target system may be, for example, a part of a semiconductor crystal. The semiconductor crystal is, for example, $Li_{24}P_4S_{20}Cl_4$.

Meanwhile, the learning end condition may include any condition related to the end of learning, for example, a condition that the estimation model which is a target mathematical model was updated a predetermined number of times, a condition that a update-induced change of the estimation model is smaller than a predetermined change, or a condition that all the labeled or unlabeled training data prepared in advance was used.

<Regarding the effects of learning processing>

As described above, the 3D wave function is used in learning. Meanwhile, the NNP technology described in Patent Literature 1 uses charge density (the sum of the squared magnitudes of one-dimensional (1D) radial wave functions from the largest occupied energy level down to the u-th (where u is an integer between 1 and U, and U is an integer of 1 or greater)) in descending order of occupied potential energy) converted to 1D information by solid angle integration, rather than the 3D wave function.

However, when there is a change in the position of the atom in 3D space, there is a change in the distribution of the wave function in 3D space. Accordingly, in the case of the learning by the learning apparatus 1 using a value having the information of the 3D wave function in the learning, the trained mathematical model considering the position information of the atom is generated. In contrast, when the charge density converted into 1D information is used instead of the 3D wave function, because it is not 3D, it is impossible to sufficiently represent the change in the distribution of the wave function in 3D space with the change in the position of the atom.

Accordingly, the learning apparatus 1 may acquire the mathematical model having higher estimation accuracy than the technology described in Patent Literature 1. Because the accuracy of energy estimation for each atom is high, the accuracy of energy estimation of the analysis target system using the trained mathematical model acquired by the learning apparatus 1 is also high.

<Example of hardware configuration of the learning apparatus 1>

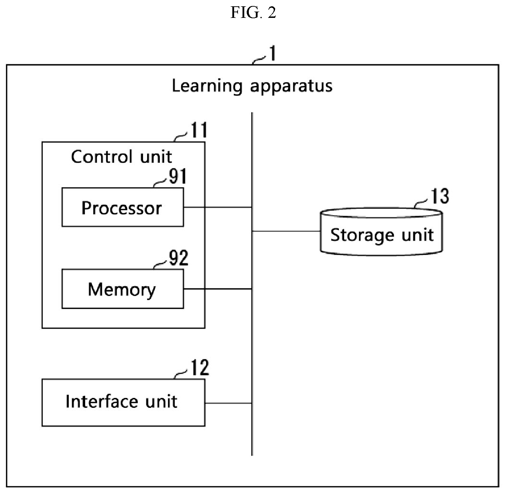

FIG. 2 is a diagram showing an example of hardware configuration of the learning apparatus 1 according to an embodiment. The learning apparatus 1 includes the control unit 11 having the processor 91 and the memory 92 connected via the bus, and executes a program. The learning apparatus 1 acts as a device including the control unit 11, an interface unit 12 and a storage unit 13 by executing the program.

More specifically, the processor 91 reads the program stored in the storage unit 13 and stores the read program in the memory 92. When the processor 91 executes the program stored in the memory 92, the learning apparatus 1 acts as the device including the control unit 11, the interface unit 12 and the storage unit 13.

The control unit 11 controls the operation of each functional unit of the learning apparatus 1. For example, the control unit 11 acquires a pair of training input data and training correct data through the interface unit 12. For example, the control unit 11 carries out learning processing. Accordingly, for example, the control unit 11 executes the estimation model. For example, the control unit 11 acquires information stored in the storage unit 13. Specifically, the processing for acquiring the information stored in the storage unit 13 is reading.

The interface unit 12 includes a communication interface to connect the learning apparatus 1 to an external device. The interface unit 12 communicates with the external device wiredly or wirelessly. The external device is, for example, a transmitter source device of the pair of training input data and training label (e.g., training correct data). In this case, the interface unit 12 acquires the pair of training input data and training label by the communication with the transmitter source device of the pair of training input data and training label.

The interface unit 12 may include an input device, for example, a mouse, a keyboard or a touch panel. The interface unit 12 may be configured as an interface connecting the input device to the learning apparatus 1. The interface unit 12 receives input of various types of information about the learning apparatus 1 wiredly or wirelessly through the input device. Meanwhile, the various types of data acquired by the communication interface of the interface unit 12 may be entered into the input device of the interface unit 12, rather than the communication interface of the interface unit 12.

The interface unit 12 outputs, for example, various types of information. The interface unit 12 includes a display device, for example, a Cathode Ray Tube (CRT) display, a liquid crystal display, or an organic Electro-Luminescence (EL) display. The interface unit 12 may be configured as an interface connecting the display device to the learning apparatus 1. For example, the display device of the interface unit 12 outputs the information entered into the communication interface or the input device of the interface unit 12.

The storage unit 13 includes a non-transitory computer-readable recording medium, for example, a magnetic hard disk or a semiconductor memory. The storage unit 13 stores various types of information about the learning apparatus 1. For example, the storage unit 13 stores various types of information generated by the operation of the control unit 11. For example, the storage unit 13 stores the information acquired by the interface unit 12.

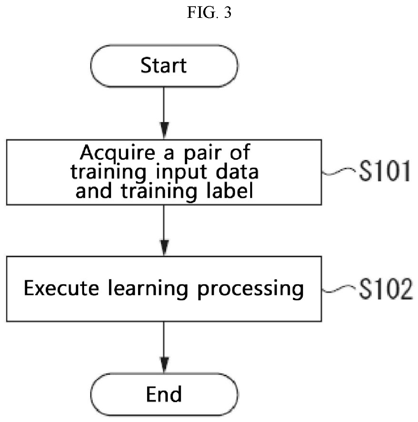

FIG. 3 is a diagram showing an example of the flow of processing executed by the learning apparatus 1 according to an embodiment. The control unit 11 acquires the pair of training input data and training label (step S101). Subsequently, the control unit 11 executes learning processing (step S102). The trained estimation model is acquired by the execution of the learning processing.

<Example of hardware configuration of the estimation apparatus 2>

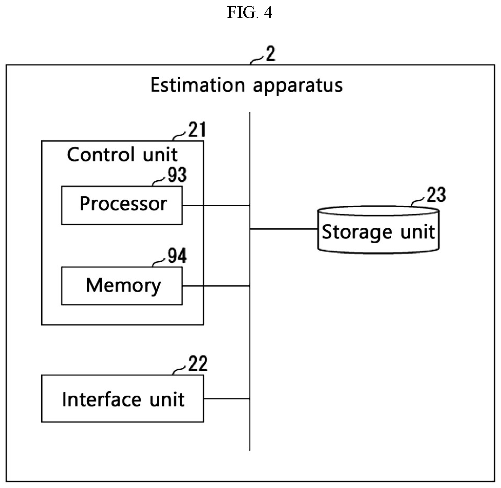

FIG. 4 is a diagram showing an example of hardware configuration of the estimation apparatus 2 according to an embodiment. The estimation apparatus 2 includes a control unit 21 (an example of an estimation unit) having a processor 93 and a memory 94 connected via a bus, and executes a program. The estimation apparatus 2 acts as a device including a control unit 21, an interface unit 22, and a storage unit 23 by executing the program.

More specifically, the processor 93 reads the program stored in the storage unit 23 and stores the read program in the memory 94. When the processor 93 executes the program stored in the memory 94, the estimation apparatus 2 acts as the device including the control unit 21, the interface unit 22 and the storage unit 23.

The control unit 21 controls the operation of each functional unit of the estimation apparatus 2. For example, the control unit 21 executes the trained estimation model. The control unit 21 acquires, for example, information input to the trained estimation model (hereinafter referred to as "estimation input data").

For example, the control unit 21 acquires the information stored in the storage unit 23. Specifically, the processing for acquiring the information stored in the storage unit 23 is reading.

The interface unit 22 includes a communication interface for connecting the estimation apparatus 2 to an external device. The interface unit 22 communicates with the external device wiredly or wirelessly. The external device is, for example, a transmitter source device of the estimation input data. In this case, the interface unit 22 acquires the estimation input data by the communication with the transmitter source device of the estimation input data. The external device is, for example, the learning apparatus 1. In this case, the interface unit 22 acquires the trained estimation model by the communication with the learning apparatus 1.

The interface unit 22 may include an input device, for example, a mouse, a keyboard or a touch panel. The interface unit 22 may be configured as an interface connecting the input device to the estimation apparatus 2. The interface unit 22 receives input of various types of information about the estimation apparatus 2 wiredly or wirelessly through the input device. Meanwhile, the various types of data acquired by the communication interface of the interface unit 22 may be entered into the input device of the interface unit 22, rather than the communication interface of the interface unit 22.

The interface unit 22 outputs, for example, various types of information. The interface unit 22 includes, for example, a display device such as a CRT display, a liquid crystal display or an organic EL display. The interface unit 22 may be configured as an interface connecting the display device to the estimation apparatus 2. For example, the display device of the interface unit 22 outputs the information entered into the communication interface or the input device of the interface unit 22.

The storage unit 23 includes a non-transitory computer-readable recording medium, for example, a magnetic hard disk or a semiconductor memory. The storage unit 23 stores various types of information about the estimation apparatus 2. For example, the storage unit 23 stores various types of information generated by the operation of the control unit 21. For example, the storage unit 23 stores the information acquired by the interface unit 22.

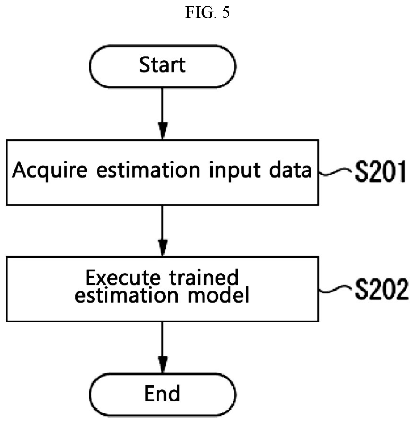

FIG. 5 is a flowchart showing an example of the flow of processing executed by the estimation apparatus 2 according to an embodiment. The control unit 21 acquires the estimation input data (step S201). Subsequently, the control unit 21 executes the trained estimation model on the acquired estimation input data (step S202). By the processing of the step S202, the result to the estimation input data is estimated. As described above, in the step S202, the control unit 21 carries out estimation using the trained estimation model.

<Experimental results>

An example of experimental results verifying the accuracy of estimation by the trained estimation model is shown. The number of wave functions used in each element was 2 (u=2). The experiment verified the estimation accuracy of each of NNP (hereinafter referred to as "first technique") that estimates the energy of the analysis target system based on a Behler-Parrinello type descriptor, NNP (hereinafter referred to as "second technique") that estimates the energy of the analysis target system based on the electron density, and the trained estimation model. In the case of using the charge density, it is a single function, i.e., u=1. Meanwhile, the second technique estimates the energy based on the electron density, but more specifically, estimates the energy based on 3D electron density by treating the electron density three-dimensionally.

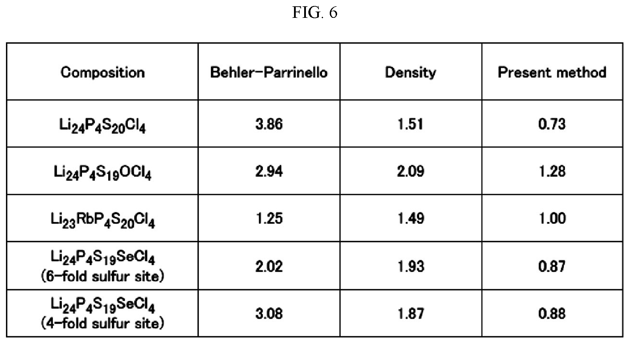

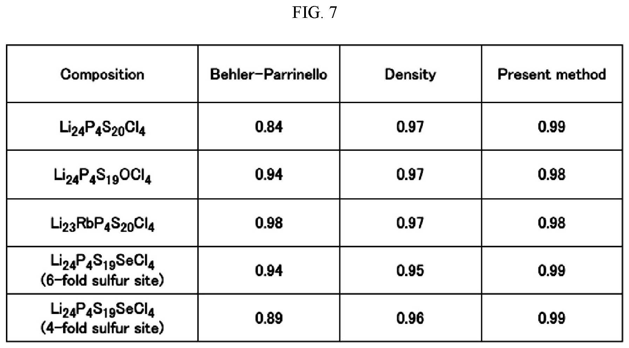

FIG. 6 is a first diagram showing an example of experimental results according to an embodiment, and FIG. 7 is a second diagram showing an example of experimental results according to an embodiment. More specifically, FIG. 6 is a diagram showing the root mean square error of the results of estimation by each of the first technique, the second technique and the trained estimation model. FIG. 7 shows the coefficient of determination $R^2$ of each of the first technique, the second technique and the trained estimation model.

In FIGS. 6 and 7, "Behler-Parrinello" indicates the first technique, "Density" indicates the second technique, and "Present method" indicates the trained estimation model. In FIGS. 6 and 7, the column "Composition" indicates the analysis target system. Accordingly, for example, the row "$Li_{24}P_4S_{20}Cl_4$" indicates that the analysis target system is $Li_{24}P_4S_{20}Cl_4$ of the semiconductor crystal.

The results in FIG. 6 show that the estimation accuracy of the trained estimation model is higher than those of the first technique and the second technique, irrespective of the analysis target system. Because higher $R^2$ indicates higher accuracy, the results of FIG. 7 show that the estimation accuracy of the trained estimation model is higher than those of the first technique and the second technique.

The learning apparatus 1 acquires the trained estimation model by the learning processing. The learning processing provides the effects described in <Regarding the effects of learning processing>. Accordingly, the learning apparatus 1 may improve the accuracy of total energy estimation by NNP.

In addition, the estimation apparatus 2 carries out estimation using the trained estimation model acquired by the learning apparatus 1. Accordingly, the estimation of total energy accuracy by NNP may be improved.

In addition, the estimation system 100 includes the learning apparatus 1. Accordingly, the accuracy of total energy estimation by NNP may be improved.

(Variation)

Meanwhile, the evaluation target feature quantity indicates the sum of 3D wave functions as described above. That is, the evaluation target feature quantity indicates the sum of wave functions at each position in 3D space. Coordinates are needed to represent each position in space. The coordinates used to represent the evaluation target feature quantity indicating the sum of 3D wave functions may be referred to as local coordinates.

The local coordinates are the set coordinates of each atom within the analysis target system, and are the coordinates with each atom as the origin. The axes of the local coordinates may include, for example, an axis (hereinafter referred to as "first axis") connecting the nearest atom of the atom at the origin to the origin, an axis (hereinafter referred to as "second axis") connecting the second nearest atom of the atom at the origin to the origin, and an axis (hereinafter referred to as "third axis") orthogonal to the first axis and the second axis. The axes of the local coordinates may be, for example, a total of three axes including a pair of orthogonal axes different from the pair of the first axis and the second axis, within the plane spanned by the first and second axes, and the third axis.

Meanwhile, each of the learning apparatus 1 and the estimation apparatus 2 may be mounted using a plurality of information processing devices connected to enable communication via a network. In this case, each processing executed by the control unit 11 and each processing executed by the control unit 21 may each be executed by the plurality of information processing devices in a distributed manner.

Meanwhile, all or some of each function of the learning apparatus 1 and the estimation apparatus 2 may be implemented using hardware, for example, an Application Specific Integrated Circuit (ASIC), a Programmable Logic Device (PLD) or a Field Programmable Gate Array (FPGA). The program may be recorded on the computer-readable recording medium. The computer-readable recording medium includes, for example, media designed to be portable and easy to move, such as flexible disk, magnetooptical disk, ROM, or CD-ROM, or storage devices such as hard disk built in a computer system. The program may be transmitted through an electrical communication line.

The embodiments of the present disclosure have been hereinabove described in detail with reference to the drawings. However, the specific configuration is not limited to the disclosed embodiment, and the present disclosure includes any other design without departing from the scope of the present disclosure.

**Claims**

1. A learning apparatus comprising:

   a control unit to carry out learning of an estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential *(u is an integer that is equal to or larger than 1 and is equal to or smaller than U, and U is an integer of 1 or greater),* and a 3D wave function of a u-th atomic orbital of each of other atoms in the system within a predetermined range of distances from the evaluation target in descending order of occupied energy potential, wherein in the learning, the estimation model is updated to reduce a difference between the sum of results of executing the estimation model for each atom in the system and the estimated energy of the system by density functional theory.

2. The learning apparatus according to claim 1,
   wherein the 3D wave function is a 3D radial wave function.

3. The learning apparatus according to claim 1,
   wherein the system is a part of a semiconductor crystal.

4. An estimation apparatus comprising:

   an estimation unit to carry out estimation by a trained estimation model acquired by a learning apparatus, the learning apparatus including a control unit to carry out learning of the estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential,

wherein in the learning, the estimation model is updated to reduce a difference between the sum of results of executing the estimation model for each atom in the system and the estimated energy of the system by density functional theory.

5. A learning method performed by a learning apparatus including a control unit to carry out learning of an estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential,

wherein in the learning, the estimation model is updated to reduce a difference between the sum of results of executing the estimation model for each atom in the system and the estimated energy of the system by density functional theory, and

wherein the learning method comprises a control step of, by the control unit, carrying out the learning of the estimation model.

6. An estimation method performed by an estimation apparatus including an estimation unit to carry out estimation by a trained estimation model acquired by a learning apparatus, the learning apparatus including a control unit to carry out learning of the estimation model which is a mathematical model for energy estimation of an analysis target atom based on an evaluation target feature quantity indicating a first sum to a u-th sum, wherein the u-th sum is a sum of a three-dimensional (3D) wave function representing a u-th atomic orbital of the evaluation target atom located in a system including one or more atoms in descending order of occupied energy potential (u is an integer that is equal to or larger than 1 and is equal to or smaller than U, and U is an integer of 1 or greater), and a 3D wave function of a u-th atomic orbital of each of other atoms in the system within a predetermined range of distances from the evaluation target in descending order of occupied energy potential,

wherein in the learning, the estimation model is updated to reduce a difference between the sum of results of executing the estimation model for each atom in the system and the estimated energy of the system by density functional theory, and

wherein the estimation method comprises an estimation step of, by the estimation unit, carrying out the estimation by the trained estimation model.

7. A program for causing a computer to act as the learning apparatus defined in any one of claims 1 to 3.

8. A program for causing a computer to act as the estimation apparatus defined in claim 4.

FIG. 1

**100**

**Learning apparatus** — 1

**Control unit** — 11

**Processor** — 91

**Memory** — 92

**Estimation apparatus** — 2

Sum of wave functions

Result of estimation by density function theory

Target mathematical model

Update learning target to reduce difference of estimation results

Estimation result

EP 4 760 728 A1

FIG. 2

FIG. 3

```
           ┌─────────────┐
           │    Start     │
           └──────┬──────┘
                  │
                  ▼
        ┌──────────────────────┐
        │  Acquire a pair of   │
        │  training input data │ ~S101
        │  and training label  │
        └──────────┬───────────┘
                   │
                   ▼
        ┌──────────────────────┐
        │ Execute learning     │ ~S102
        │ processing           │
        └──────────┬───────────┘
                   │
                   ▼
           ┌─────────────┐
           │     End      │
           └─────────────┘
```

FIG. 4

FIG. 5

FIG. 6

| Composition | Behler-Parrinello | Density | Present method |
|---|---|---|---|
| $Li_{24}P_4S_{20}Cl_4$ | 3.86 | 1.51 | 0.73 |
| $Li_{24}P_4S_{19}OCl_4$ | 2.94 | 2.09 | 1.28 |
| $Li_{23}RbP_4S_{20}Cl_4$ | 1.25 | 1.49 | 1.00 |
| $Li_{24}P_4S_{19}SeCl_4$ (6-fold sulfur site) | 2.02 | 1.93 | 0.87 |
| $Li_{24}P_4S_{19}SeCl_4$ (4-fold sulfur site) | 3.08 | 1.87 | 0.88 |

FIG. 7

| Composition | Behler-Parrinello | Density | Present method |
|---|---|---|---|
| $Li_{24}P_4S_{20}Cl_4$ | 0.84 | 0.97 | 0.99 |
| $Li_{24}P_4S_{19}OCl_4$ | 0.94 | 0.97 | 0.98 |
| $Li_{23}RbP_4S_{20}Cl_4$ | 0.98 | 0.97 | 0.98 |
| $Li_{24}P_4S_{19}SeCl_4$ (6-fold sulfur site) | 0.94 | 0.95 | 0.99 |
| $Li_{24}P_4S_{19}SeCl_4$ (4-fold sulfur site) | 0.89 | 0.96 | 0.99 |

## INTERNATIONAL SEARCH REPORT

| International application No. |
| --- |
| **PCT/KR2025/003954** |

| A. | CLASSIFICATION OF SUBJECT MATTER |
| --- | --- |

**G16C 10/00**(2019.01)i; **G16C 20/70**(2019.01)i; **G16C 60/00**(2019.01)i; **G06N 3/08**(2006.01)i

According to International Patent Classification (IPC) or to both national classification and IPC

| B. | FIELDS SEARCHED |
| --- | --- |

Minimum documentation searched (classification system followed by classification symbols)

G16C 10/00(2019.01); G06N 20/00(2019.01); G06N 3/02(2006.01); G06N 5/02(2006.01); G06N 5/04(2006.01); G16C 20/50(2019.01); G16C 20/70(2019.01); G16C 60/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 뉴럴 네트워크 포텐셜(neural network potential), 원자(atom), 궤도 (orbit), 점유 준위 에너지(occupied level energy), 파동 함수(wave function), 밀도범함수법(density functional method), 프로세서(processor), 메모리(memory), 학습장치(learning device), 추정장치(inference device), 컴퓨터 (computer), 프로그램(program)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2022-189626 A (ENEOS CORP. et al.) 22 December 2022 (2022-12-22) <br> abstract, claim 1, paragraph [0006], figure 1 | 1-8 |
| A | WO 2021-243106 A1 (CALIFORNIA INSTITUTE OF TECHNOLOGY et al.) 02 December 2021 (2021-12-02) <br> entire document | 1-8 |
| A | WO 2022-212299 A1 (CARNEGIE MELLON UNIVERSITY et al.) 06 October 2022 (2022-10-06) <br> entire document | 1-8 |
| A | US 2023-0095631 A1 (ROBERT BOSCH GMBH) 30 March 2023 (2023-03-30) <br> entire document | 1-8 |

[✓] Further documents are listed in the continuation of Box C.　　[✓] See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **07 July 2025** | **08 July 2025** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2025/003954**

**C.     DOCUMENTS CONSIDERED TO BE RELEVANT**

| Category* | Citation of document, with indication, where appropriate, of the relevant passages | Relevant to claim No. |
|-----------|-----------|-----------|
| A | US 2022-0207393 A1 (SAMSUNG ELECTRONICS CO., LTD.) 30 June 2022 (2022-06-30)<br>entire document | 1-8 |

Form PCT/ISA/210 (second sheet) (July 2022)

**INTERNATIONAL SEARCH REPORT**
Information on patent family members

International application No.

**PCT/KR2025/003954**

| Patent document cited in search report | | | Publication date (day/month/year) | Patent family member(s) | | | Publication date (day/month/year) |
|---|---|---|---|---|---|---|---|
| JP | 2022-189626 | A | 22 December 2022 | | None | | |
| WO | 2021-243106 | A1 | 02 December 2021 | CN | 115836351 | A | 21 March 2023 |
| | | | | EP | 4158640 | A1 | 05 April 2023 |
| | | | | US | 2022-0165364 | A1 | 26 May 2022 |
| WO | 2022-212299 | A1 | 06 October 2022 | US | 2024-0304285 | A1 | 12 September 2024 |
| US | 2023-0095631 | A1 | 30 March 2023 | CN | 115862748 | A | 28 March 2023 |
| | | | | US | 12094580 | B2 | 17 September 2024 |
| US | 2022-0207393 | A1 | 30 June 2022 | KR | 10-2022-0097594 | A | 08 July 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024056799 A

- US 20220207393 A

**Non-patent literature cited in the description**

- **J. BEHLER** ; **M. PARRINELLO**. Generalized Neural-Network Representation of High-Dimensional Potential-Energy Surfaces. *Phys. Rev. Lett.*, 2007, vol. 98, 146401
- **AP BARTOK et al.** Gaussian Approximation Potentials: The Accuracy of Quantum Mechanics, without the Electrons. *Phys. Rev. Lett. Vol.*, 2010, vol. 104, 136403

- **AP THOMPSON et al.** Spectral neighbor analysis method for automated generation of quantum-accurate interatomic potentials.. *Journal of Computational Physics*, 2015, vol. 285, 316-330